# EUROPÄISCHE PATENTANMELDUNG

(11) **EP 2 559 411 A1**
(43) Veröffentlichungstag der Anmeldung: **20.02.2013**
(21) Anmeldenummer: 12179854.0
(22) Anmeldetag: 09.08.2012
(51) Int. Cl.: A61F 7/02, A61F 7/08

(54) **Wärmespendendes Produkt**

(30) Priorität: 19.08.2011 DE 102011052851; 19.08.2011 DE 102011052854
(71) Anmelder: Leifheit AG, 56377 Nassau (DE)
(72) Erfinder: Stange, Dr., Pedro, 65582 Diez (DE)
(74) Vertreter: Bungartz, Klaus Peter

(57) **Zusammenfassung**

Die Erfindung betrifft ein wärmespendendes Produkt mit Wärmespeicher (1) und Vorrichtung zur Temperatureinstellung mit wenigstens einer aktiven Oberfläche des Wärmespeichers (1) zum Erwärmen einer Fläche, insbesondere eines Untergrundes oder einer Person. Solche, zum Beispiel als Wärmeflasche bekannten Produkte haben den Nachteil, dass sie zu Beginn oft zu heiß sind und sich die Temperatur nicht wunschgemäß einstellen lässt.

Dies verbessert die Erfindung dadurch, dass der Wärmespeicher (1) zumindest eine wärmeisolierende Abdeckschicht (2) mit einer ersten Wärmeleitfähigkeit aufweist, die zur Reduktion des Wärmeübergangs von dem Wärmespeicher (1) auf die Fläche auf die aktive Oberfläche des Wärmespeichers (1) auflegbar ist.

## Beschreibung

### Technisches Gebiet

Die Erfindung betrifft ein wärmespendendes Produkt mit Wärmespeicher und einer Vorrichtung zur Temperatureinstellung mit wenigstens einer wärmeabgebenden Oberfläche des Wärmespeichers zum Erwärmen einer Fläche, insbesondere eines Untergrundes oder einer Person.

### Stand der Technik

Wärmespendende Produkte mit Wärmespeicher und einer Temperatureinstellung dieser Art sind als aktive oder passive Wärmekissen aus der US 5 891 187 A bekannt. Bei diesem Produkt handelt es sich um eine wärmeabgebende Unterlage für einen Patienten, der während einer Behandlung auf der Unterlage liegt, die zur Verringerung von Wärmeverlusten nach oben Decken aufweist, die an der Unterlage befestigt sind und über den liegenden Patienten geschlagen werden können, so dass die Wärme nicht so einfach nicht am Patienten vorbei nach oben abströmen kann.

Die passiven Wärmespeicher haben den Nachteil, dass ihre Temperatur, nachdem sie einmal aufgeheizt worden, nicht mehr veränderbar ist. Vielmehr muss der Benutzer im Falle eines zu heißen Wärmespeichers so lange warten, bis sich der Wärmespeicher auf die gewünschte Temperatur abgekühlt hat. Die in diesem Dokument auch beschriebenen aktiven Wärmespeicher, zum Beispiel mit elektrischer Heizung, haben aber den Nachteil, dass sie zum einen netzabhängig sind und neben der aktiven Wärmeerzeugung auch eine vergleichsweise aufwändige Temperaturregelung benötigen.

Aus der DE 20 2009 013 565 U1 ist eine Pferdedecke mit einem passiven Wärmekissen bekannt, das den Sattel von unten anwärmt. Dieses Wärmekissen weist den gleichen Nachteil auf, die das oben beschriebene, passive Wärmekissen für die Patientenwarmhaltung.

Aus der US 7 319 207 ist wiederum eine aktive Wärmequelle bekannt, die aber ebenfalls wegen der Netzabhängigkeit und der Regelung aufwändig ist und kaum mobil einsetzbar ist.

### Kurzbeschreibung der Erfindung

Aufgabe der Erfindung ist es, ein kostengünstiges wärmespendendes Produkt zu schaffen, bei dem auf leichte und effiziente Weise auch ohne aufwändige Regelung die abgegebene Wärme eingestellt werden kann und das mobil ohne Netzabhängigkeit möglichst angenehm und variabel verwendet werden kann.

Diese Aufgabe wird nach der Erfindung dadurch gelöst, dass der Wärmespeicher zumindest eine wärmeisolierende Abdeckschicht mit einer ersten Wärmeleitfähigkeit aufweist, die zur Reduktion des Wärmeübergangs von dem Wärmespeicher auf die zu erwärmende Fläche auf die aktive Oberfläche des Wärmespeichers auflegbar ist.

Bei einer bevorzugten Ausgestaltung ist der Wärmespeicher ein passiver Wärmespeicher, der zum Beispiel als Latentwärmespeicher ausgebildet sein kann und in der Mikrowelle oder einem Ofen erwärmt wird. Die Erfindung ist aber auch auf alle anderen Formen von Wärmespeichern anwendbar, selbst in Verbindung mit elektrisch betriebenen Wärmespeichern kann die Erfindung verwendet werden.

Erfindungsgemäß ist eine wärmeisolierende Abdeckschicht vorgesehen, die über die aktive Oberfläche des Wärmespeichers gelegt werden kann. Diese aktive Oberfläche des Wärmespeichers ist zum Beispiel die Oberfläche eines wärmeabgebenden Heizkissens. Die wärmeisolierende Abdeckschicht ist dabei als isolierende Schicht ausgebildet, die einen Teil der Wärme des Heizkissens zurückhält. Dies hat den besonderen Vorteil, dass bei zu heißem Heizkissen über die thermische Isolation die Temperatur, die auf die Unterlage bzw. die Person einwirkt reduziert werden kann. Lässt die Heizwirkung nach, beispielsweise weil das Heizkissen wieder abkühlt, kann dann die wärmeisolierende Abdeckschicht wieder entfernt werden, so dass sich hierüber eine Temperatursteigerung erzielen lässt.

Bei einer weiteren bevorzugten Ausgestaltung der Erfindung werden zwei oder mehr wärmeisolierende Abdeckschichten verwendet, die alternativ oder kumulativ verwendet werden können. Bei der alternativen Verwendung können zum Beispiel die wärmeisolierenden Abdeckschichten an unterschiedlichen Rändern des Heizkissens befestigt sein. In diesem Fall kann der Benutzer jeweils eine gewünschte Abdeckschicht auf die aktive Oberfläche des Heizkissens legen. Diese Ausgestaltung weist den zusätzlichen Vorteil auf, dass nicht nur eine alternative sondern auch die kumulative Verwendung möglich ist.

Vorteilhaft kann ferner sein, wenn die Abdeckschichten längs eines Umschlagrandes mit dem Heizkissen verbunden sind. Dieser Umschlagrand verläuft zum Beispiel längs eines äußeren Randes des Heizkissens. An diesem äußeren Rand ist dann die Abdeckschicht mit einem ihrer Ränder befestigt, wobei dies über eine Naht oder auch über eine lösbare Verbindung, zum Beispiel einen Klettverschluss erfolgen kann. Um diesen Umschlagrand kann die Abdeckschicht dann entweder auf die Vorderseite oder die Rückseite des Heizkissens geklappt werden, so dass sie entweder die aktive Oberfläche freigibt oder bedeckt.

Bevorzugt weist die wärmeisolierende Abdeckschicht ein Befestigungsmittel auf, über das sie auf der Rückseite des Heizkissens oder der aktiven Oberfläche befestigt werden kann. Sofern mehrere wärmeisolierende Abdeckschichten vorgesehen sind, die längst unterschiedlicher Umschlagränder umgeklappt werden können, können auch die wärmeisolierenden Abdeckschichten untereinander, zum Beispiel über Klettverschlüsse, verwendbar ausgebildet sein, so dass die jeweils äußeren Abdeckschichten auf der darunter liegenden Abdeckschicht befestigt werden kann.

Insbesondere dann, wenn die wärmeisolierenden Abdeckschichten unterschiedliche Wärmeleitfähigkeiten aufweisen, ist es sinnvoll, dass diese unterschiedlichen thermischen Eigenschaften über eine farbliche Markierung gekennzeichnet wird. So können zum Beispiel die unterschiedlichen Abdeckschichten andere Farben aufweisen. Auch können Werte für den Grad der thermischen Isolation aufgedruckt werden bzw. diese Aufdrucke in Verbindung mit der Farbkodierung kombiniert werden.

Sofern die wärmeisolierenden Abdeckschichten längs eines gemeinsamen Randes des Heizkissens mit diesem verbunden sind, können diese Abdeckschichten nacheinander von vorne nach hinten geklappt werden. Hier können sowohl auf der aktiven Oberfläche als auch auf der Rückseite des Wärmekissen Befestigungsmittel vorgesehen sein, wobei im Falle mehrerer Abdeckschichten diese wiederum entweder unabhängig voneinander an dem Heizkissen oder im Falle der äußeren Abdeckschichten an der darunter liegenden Abdeckschicht befestigt werden können.

Auf der, der aktiven Oberfläche gegenüberliegenden Seite weist das Heizkissen im Falle einer weiteren bevorzugten Ausgestaltung eine thermische Isolation auf. Diese thermische Isolation bewirkt, dass die Wärme nur in Richtung der aktiven Oberfläche abgegeben wird bzw. dass ein Großteil der Wärme in diese Richtung abgegeben werden kann. Die thermische Isolation kann zum Beispiel von einer wärmereflektierenden Schicht gebildet sein, wobei auch andere Isolatoren zum Einsatz kommen können.

Eine weitere bevorzugte Ausgestaltung der Erfindung verwendet einen hüllenartigen Überzug als wärmeisolierende Abdeckschicht. In diesen Überzug kann dann das Heizkissen eingesteckt werden, das auch hier eine aktive Oberfläche und eine Rückseite aufweist, wobei diese Rückseite mit einer thermischen Isolation versehen sein kann. Im Falle einer besonders bevorzugten Ausgestaltung weist die Hülle zwei unterschiedlich ausgebildete Seiten auf, wobei eine Vorderseite eine erste Wärmeleitfähigkeit und eine Rückseite eine zweite Wärmeleitfähigkeit besitzt.

Sofern nun der Überzug relativ zum Heizkissen verdreht wird, kommt jeweils eine der beiden Seiten, entweder die Vorderseite oder die Rückseite, vor der aktiven Oberfläche zum liegen, so dass der Benutzer nur durch Drehen der Hülle die thermischen Eigenschaften der Abdeckschicht ändern kann. Sofern gar keine Abdeckschicht gewünscht ist, kann der Benutzer den Überzug auch vollständig entfernen.

Schließlich kann es günstig sein, wenn der Wärmespeicher auf einer Seite eine thermische Isolation aufweist, während er eine thermisch gering isolierte zweite Seite aufweist, die die wärmeabgebende Oberfläche bildet. Auf diese Weise wird die Wärme dorthin geleitet, wo sie bestimmungsgemäß benötigt wird. Ferner bleibt die gegenüberliegende Seite, die üblicherweise ja nicht zum Aufwärmen des Körpers benutzt werden kann, kühl, was zum einen hinsichtlich des Anfassens des Wärmespeichers günstig ist und zum anderen verhindert, dass beispielsweise empfindliche Textilien beschädigt werden.

Besonders vorteilhaft ist es, wenn der hüllenartige Überzug mit der oben beschriebenen Wärmeisolierung des Wärmespeichers kombiniert wird. So kann zum Beispiel der hüllenartige Überzug zwei gegenüberliegende Seiten aufweisen, die unterschiedlich stark thermisch isoliert sind. Sofern dies beim Wärmespeicher ebenfalls der Fall ist, kann durch unterschiedliche Kombinationen die Heizwirkung der Ansicht gleicher Energiebeladung des Wärmespeichers vom Benutzer eingestellt werden. Hierzu kann er den Überzug in verschiedenen Orientierungen über den Wärmespeicher ziehen und dann die gewünschte Fläche zum erwärmen des Körpers verwenden.

Ist zum Beispiel der Wärmespeicher noch recht heiß, kann er die Seite des Wärmespeichers, die thermisch besser isoliert ist, zunächst mit einer stark isolierenden Außenseite des Überzugs kombinieren, um so die maximale Isolation zu erzielen. Lässt die Heizwirkung nach oder wünscht der Benutzer eine größere Wärmeabgabe, kann er entweder den Überzug oder den Wärmespeicher drehen, so dass die Wärme abgebende Fläche dann weniger stark isoliert ist. Zur Erzielung der maximalen Wärmeabgabe kann er dann diejenige Fläche des Wärmespeichers, die nur sehr gering isoliert ist mit dem Wandabschnitt des Überzugs kombinieren, der ebenfalls am geringsten isoliert ist.

Der Überzug wiederum kann mehrschichtig ausgebildet sein. So kann zum Beispiel das äußere Material des Überzuges von einem Stoff gebildet sein. In diesem Fall ähnelt dieser Überzug einer Kissenhülle. Im Inneren kann dann eine Innenhülle vorgesehen sein, die relativ zum Stoff vertretbar oder festgelegt ist. Dieser kann zum Beispiel an einer Seite eine metallische Folie zur Wärmereflektion aufweisen. Auch eine übliche thermische isolierte Schicht, zum Beispiel von einer Luft gefüllten, porösen Struktur kann hier Verwendung finden oder mit der metallischen Folie kombiniert werden. Schließlich ist es möglich, dass die Kissenhülle einseitig eine Einstiegvorrichtung für die thermische Isolation aufweist.

Auf Seiten der minimalen thermischen Isolation können Stoffe verwendet werden, die die Wärmeleitung verbessern. Hier können zum Beispiel Metallfäden in den Stoff mit eingewilligt werden, die die Wärmeleitung des Stoffes des Überzuges vergrößern.

Ein weiteres bevorzugte Merkmal ist ein Thermoindikator, der auch ohne die Abdeckschichten sinnvoll verwendet werden kann: Das wärmespendende Produkt weist dabei den Wärmespeicher auf, der durch Energiezufuhr mit Wärmeenergie beladbar ist und anschließend die Wärmeenergie über zumindest eine wärmeabgebende Oberfläche abzugeben vermag, wobei ein Wärmeindikator vorgesehen ist, der derart mit dem Wärmespeicher zusammenwirkt, dass er die Temperatur des Wärmespeichers anzuzeigen vermag. Dies kann dadurch geschehen, dass der Wärmeindikator derart mit dem Wärmespeicher verbunden ist, dass er die Temperatur des Wärmespeichers annimmt und optisch wiedergibt. Hierzu kann der Wärmeindikator so ausgebildet sein, dass er bei Unterschreiten und Überschreiten einer definierten Temperaturgrenze jeweils seine Erscheinungsweise, insbesondere seine Farbe ändert.

Der Wärmeindikator weist bei einer bevorzugten Ausgestaltung einen thermochromen Farbstoff auf, der unter Ausschluss von Luft und Feuchtigkeit im Wärmeindikator eingekapselt ist.

Bevorzugt kann der Wärmeindikator zumindest einen Bereich aufweisen, der von einer oberen Schicht und einer unteren Schicht gebildet ist, zwischen denen eine Substanz eingeschlossen ist, die die unterhalb der Grenztemperatur undurchsichtig und oberhalb der Grenztemperatur transparent oder durchsichtig ist, wobei die obere Schicht transparent und die untere Schicht farbig oder schwarz ist, wobei die Substanz beim Erwärmen transparent oder durchsichtig und beim Abkühlen reversibel undurchsichtig wird. Hierbei können die obere Schicht und die untere Schicht von Kunststofffolien, insbesondere aus Polyolefin oder Polyvenylchlorid gebildet sein, wobei die Substanz von einem Kohlenwasserstoff gebildet sein kann. In Betracht kommt hier zum Beispiel ein gesättigter, ungesättigter, cyclischer, verzweigter oder Isopren basierender Kohlenwasserstoff oder eine Mischung hieraus ist. Auch kann der Kohlenwasserstoff Parrafinwachs, insbesondere mit einem Schmelzpunkt zwischen 40° und 80°C, sein.

Der Wärmeindikator kann an dem Wärmespeicher selbst, aber auch oder zusätzlich an einer oder allen Abdeckschichten angeordnet sein. Der Wärmespeicher kann eine vergleichsweise feste Form mit einer kaum änderbaren Kontur aufweisen oder auch weich, zum Beispiel als Kissen oder zusammenrollbare Decke ausgebildet sein.

Weitere Merkmale und Vorteile der Erfindung ergeben sich aus den Unteransprüchen und aus der nachfolgenden Beschreibung bevorzugter Ausführungsbeispiele anhand der Zeichnungen.

### Kurze Beschreibung der Zeichnungsfiguren

In den Zeichnungen zeigt:

Figur 1 eine erste Ausgestaltung der Erfindung,

Figur 2 den Wärmespeicher einer zweiten Ausgestaltung der Erfindung,

Figur 3 einen hüllenartigen Überzug für den Wärmespeicher aus Figur 2,

Figur 4 in den in Figur 2 dargestellten Wärmespeicher, über den der in Figur 3 dargestellte Überzug in einer ersten Orientierung gezogen ist und

Figur 5 den in Figur 2 dargestellten Wärmespeicher, über den der in Figur 3 dargestellte Überzug in einer zweiten Orientierung gezogen ist.

### Beschreibung der Ausführungsarten

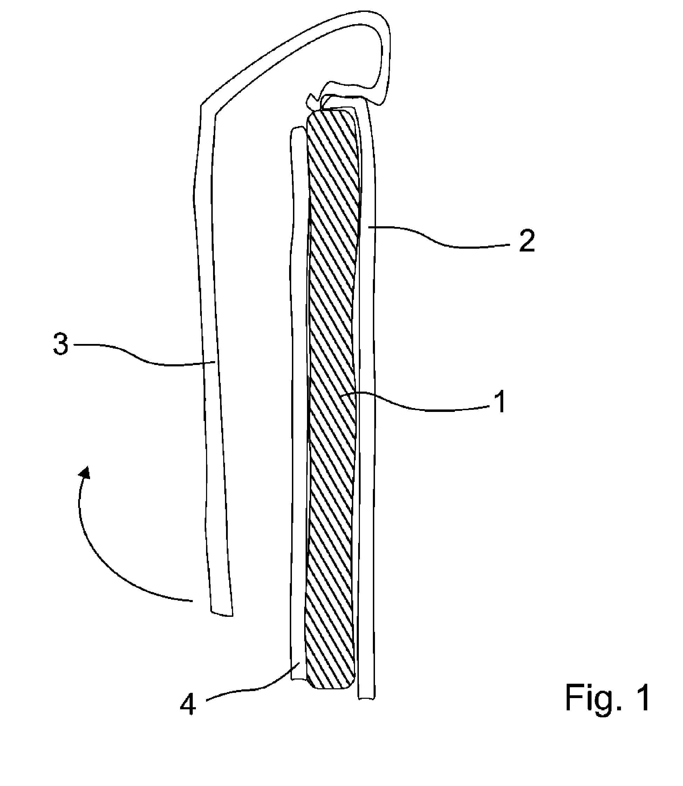

In Figur 1 ist eine erste Ausgestaltung der Erfindung dargestellt.. Das wärmespendende Produkt wird von einem Heizkissen gebildet, das hier als Wärmespeicher 1 dient. Im oberen Bereich ist am linken Längsrand des Wärmespeichers 1 der Umschlagrand vorgesehen. Hier sind zwei wärmeisolierende Abdeckschichten 2, 3 befestigt, wobei der Wärmespeicher 1 in der dargestellten Position auf der rechten Seite die aktive Oberfläche aufweist.

Um die Heizwirkung zu reduzieren kann der Benutzer die erste wärmeisolierende Abdeckschicht 2, wie in der gezeigten Stellung dargestellt, vor die aktive Oberfläche legen. Sollte die thermische Isolation der ersten wärmeisolierenden Abdeckschicht 2 nicht ausreichen, kann er die in der dargestellten Stellung noch nach hinten geklappte zweite wärmeisolierende Abdeckschicht 3 ebenfalls noch nach vorne klappen.

Das Heizkissen ist an seiner Rückseite hier mit einer thermischen Isolation 4 versehen. Diese thermische Isolation deckt die Rückseite ab und verhindert so, dass die Wärme des Heizkissens ungewollt in die Umgebung abgegeben wird. Gleichzeitig wird die Wärme in Richtung der aktiven Oberfläche gelenkt. Zusätzlich hat die thermische Isolation der Vorteil, dass eine der beiden Oberflächen des Heizkissens vergleichsweise kühl bleibt, so dass das Heizkissen an dieser Seite angefasst werden kann oder auf einer wärmeempfindlichen Oberfläche abgelegt werden kann. Um das Anfassen weiter zu erleichtern, können auch die äußeren Ränder des Heizkissens mit einer thermischen Isolation versehen sein.

Wie zu erkennen ist, kann der Benutzer durch die beiden Abdeckschichten hier fünf verschiedenen Wärmemodi einstellen: Einmal kann der den Wärmespeicher direkt an seinen Körper oder die sonstige, zu wärmende Fläche halten. Dann erhält er die geringste Isolation und den maximalen Wärmeübergang. Dann kann er die ersten und ggfs. auch die zweite Abdeckschicht zwischenlegen, indem er diese umschlägt und so vor den Wärmespeicher anordnet, so dass zwei Isolationswerte zur Verfügung stehen. Auf gleiche Weise kann er die Abdeckschichten auch auf der anderen, thermisch im Vergleich zur Vorderseite anders ausgebildeten Seite des Wärmespeichers einzeln oder zusammen einsetzen.

Sind die Abdeckschichten an unterschiedlichen Seiten des Wärmespeichers angeordnet und mit diesem befestigt, kann der Benutzer sogar wählen, ob er beide Abdeckschichten alternativ oder kumulativ einsetzen möchte, was die Anzahl der Betriebsmodi, die durch eine unterschiedliche Isolationswirkung gekennzeichnet sind, sogar auf acht Modi erhöht:

Modus 1: Keine Abdeckschicht zwischen zu wärmender Fläche und dem Wärmespeicher mit an die zu wärmende Fläche angelegter Vorderseite.

Modus 2: Keine Abdeckschicht zwischen zu wärmender Fläche und dem Wärmespeicher mit an die zu wärmende Fläche angelegter Rückseite.

Modus 3: Die erste Abdeckschicht zwischen zu wärmender Fläche und dem Wärmespeicher mit an die zu wärmende Fläche angelegter Vorderseite.

Modus 4: Die erste Abdeckschicht zwischen zu wärmender Fläche und dem Wärmespeicher mit an die zu wärmende Fläche angelegter Rückseite.

Modus 5: Die zweite Abdeckschicht zwischen zu wärmender Fläche und dem Wärmespeicher mit an die zu wärmende Fläche angelegter Vorderseite.

Modus 6: Die zweite Abdeckschicht zwischen zu wärmender Fläche und dem Wärmespeicher mit an die zu wärmende Fläche angelegter Rückseite.

Modus 7: Die erste Abdeckschicht und die zweite Abdeckschicht zwischen zu wärmender Fläche und dem Wärmespeicher mit an die zu wärmende Fläche angelegter Vorderseite.

Modus 8: Die erste Abdeckschicht und die zweite Abdeckschicht zwischen zu wärmender Fläche und dem Wärmespeicher mit an die zu wärmende Fläche angelegter Rückseite.

Es ist offensichtlich, dass diese Vielfalt durch weitere (zueinander in der thermischen Wirkung identische oder unterschiedliche) Abdeckschichten, durch die Verwendung auch der Seitenflächen mit jeweils anderer Wärmeabgabe oder ähnliche Maßnahmen weiter und einfach erhöht werden kann, sofern dies gewollt ist.

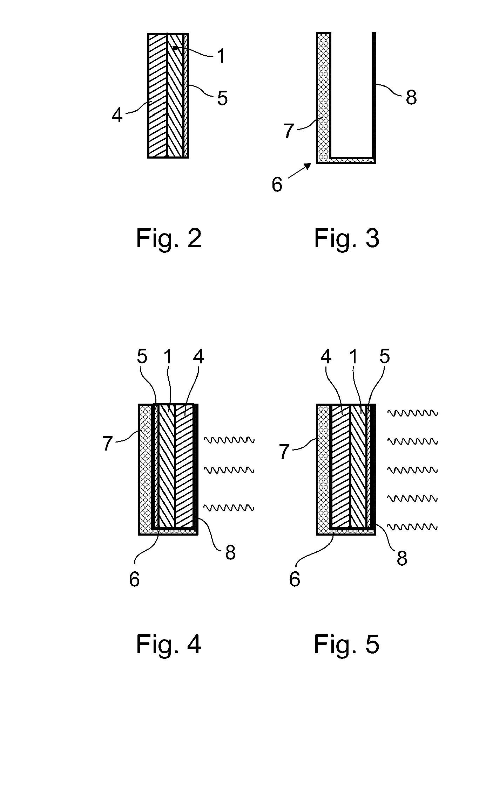

Die Figuren 2-5 zeigen eine weitere Ausgestaltung der Erfindung. Hier ist ein Wärmespeicher 1 als Kern vorgesehen, der zum Beispiel in einer Mikrowelle mit Energie beladen werden kann. Der Wärmespeicher 1 ist in Figur 2 dargestellt. Die rechte Seite 5 des Wärmespeichers 1 ist mit einer thermisch gering wirkenden, isolierenden Schicht versehen. Die gegenüberliegende Seite ist dagegen mit einer thermischen Isolation 4 versehen, die dafür Sorge trägt, dass die vom Wärmespeicher 1 abgegebene Wärmeenergie in Richtung der thermisch gering isolierten Seite 5 reflektiert und von dieser dann an die Umgebung abgegeben werden kann. Der dargestellte Wärmespeicher 1 weist also eine warme Seite und einem Vergleich zur warmen Seite eher kühlere Seite auf.

In Figur 3 ist als Zubehörteil für den Wärmespeicher 1 ein hüllenartiger Überzug 6 schematisch dargestellt. Dieser Überzug 6 kann als festes Bauteil mit einem Einschub ausgebildet sein, in den der Wärmespeicher 1 einsetzbar ist. Alternativ kommt auch eine flexible Ausgestaltung nach der Art einer Kissenhülle in Betracht. Letztlich ist für den Überzug 6 und seine erfindungsgemäße Funktion maßgeblich, dass der Überzug 6 einen Wärmeleitabschnitt 7 und einen Isolierabschnitt 8 aufweist, wobei der Wärmeleitabschnitt 7 eine geringe thermische Isolation bewirkt, während der Isolierabschnitt 8 eine hohe Isolationswirkung besitzt.

In Verbindung mit dem Wärmeleitabschnitt 7 und dem Isolierabschnitt 8 soll unter dem Begriff Abschnitt verstanden sein, dass es sich hierbei um einen Teilbereich der Wandung des Überzugs 6 handelt. Üblicherweise wird dies eine vollständige Seite des Überzugs 6 sein, so dass also einer der beiden Breitseiten des Überzugs 6 den Wärmeleitabschnitt 7 und die gegenüberliegende Breitseite den Isolierabschnitt 8 bilden.

Es ist jedoch ebenso gut möglich, dass auch nur Teilbereiche der Seitenwandung die Funktionsbereiche Wärmeleitabschnitt 7 und Isolierabschnitt 6 bilden. Grundsätzlich könnten diese beiden Funktionsbereiche sogar auf einer gemeinsamen Seite angeordnet sein, wenn zum Beispiel der Wärmespeicher 1 innerhalb einer Aufnahme des Überzugs quer verschiebbar ist. Ferner kann der Wärmespeicher auch auf einer Seite mit einer thermisch durchlässigen und einer thermisch isolierenden Schicht versehen sein, so dass zum Beispiel durch ein anderes Anordnen des Wärmespeichers 1 relativ zum Überzug 6 der Grad der durchgelassenen Wärme des Wärmespeichers eins einstellbar ist.

Besonders bevorzugt ist jedoch eine einfache und möglichst nicht über die benötigte wärmeabgebende Fläche räumlich nicht hinausgehende Ausgestaltung des wärmespendenden Produkts. Diese weist den Wärmespeicher 1 auf, der einen Seite wegen der thermischen Isolation 4 vergleichsweise wenig Wärme abgibt, während er auf der gegenüberliegenden Seite aufgrund der thermisch gering isolierten Seite 5 den Hauptteil seiner Wärme abgibt. Dieser Wärmespeicher 1 ist dann in den hüllenartigen Überzug 6 eingesteckt, der ebenfalls auf einer Seite den Wärmeleitabschnitt 7 und auf der gegenüberliegenden Seite den Isolierabschnitt 8 aufweist.

Bei dieser Kombination kann der Benutzer durch wechselseitiges Einstecken des Wärmespeichers 1 entweder die warme Seite oder die kühlere Seite des Wärmespeichers 1 neben dem Wärmeleitabschnitt 7 oder dem Isolierabschnitt 8 platzieren. Nun kann er eine der beiden Seiten des Überzugs 6, also entweder in Wärmeleitabschnitt 7 oder die Isolierabschnitt 8, als wärmespendende Oberfläche nutzen. Auf diese Weise kann durch einfaches Umdrehen der beiden Komponenten des Produktes bei gleicher Energiebeladung des Wärmespeichers 1 zwischen vier verschiedenen Temperaturen an der wärmespendenden Oberfläche ausgewählt werden. Die beiden Orientierungen des Wärmespeichers 1 relativ zum Überzug 6 sind in den Figuren 4 und 5 dargestellt.

Selbstverständlich sind weitere Lösungen denkbar, insbesondere könnte zum Beispiel eine zusätzliche isolierte Schicht zwischen den Wärmespeicher 1 und den hüllenartigen Überzug 6 an dessen Innenseite vorgesehen werden. Auch können die oben beschriebenen Lösungen miteinander kombiniert werden, d.h. der hüllenartige Überzug 6 kann in Kombination mit einem Wärmespeicher 1 Verwendung finden, der seinerseits wiederum eine erste und gegebenenfalls auch zweite wärmeisolierende Abdeckschicht 2, 3 aufweist, wobei in diesem Fall die erste wärmeisolierende Abdeckschicht 2 und, falls vorhanden, die zweite wärmeisolierende Abdeckschicht 3 so ausgebildet sind, dass sie entweder zusammen mit den Wärmespeicher 1 in den hüllenartigen Überzug 6 einsteckbar sind oder auf der Außenseite des Überzugs 6 um den Überzug 6 herum gelegt werden können.

### Liste der Bezugszeichen

**Bezugszeichenliste:**

1 Wärmespeicher

2 Erste wärmeisolierende Abdeckschicht

3 Zweite wärmeisolierende Abdeckschicht

4 Thermische Isolation

5 Thermisch gering isolierte Seite des Wärmespeichers

6 Hüllenartiger Überzug

7 Wärmeleitabschnitt des hüllenartigen Überzugs

8 Isolierabschnitt des hüllenartigen Überzugs

## Patentansprüche

1. Wärmespendendes Produkt mit einem Wärmespeicher (1), der wenigstens eine wärmeabgebende Oberfläche zum Erwärmen einer Fläche, insbesondere eines Untergrundes oder einer Person aufweist, und mit einer Vorrichtung zur Temperatureinstellung, **dadurch gekennzeichnet, dass** der Wärmespeicher (1) zur Reduktion des Wärmeübergangs von dem Wärmespeicher (1) auf die Fläche zumindest eine wärmeisolierende Abdeckschicht (2) mit einer ersten Wärmeleitfähigkeit aufweist, die auf die wärmeabgebende Oberfläche des Wärmespeichers (1) auflegbar ist.

2. Wärmespendendes Produkt mit Wärmespeicher (1) und Temperatureinstellung nach Anspruch 1, **dadurch gekennzeichnet, dass** der Wärmespeicher (1) als Heizkissen ausgebildet ist, wobei die wärmeisolierende Abdeckschicht (2) an einer Seite des Wärmespeichers (1) mit diesem längs eines Umschlagrandes verbunden ist, der von einem der Ränder des Heizkissens gebildet ist.

3. Wärmespendendes Produkt mit Wärmespeicher (1) und Temperatureinstellung nach Anspruch 1 oder 2, **dadurch gekennzeichnet, dass** der Wärmespeicher (1) eine, die wärmeabgebende Oberfläche bildende erste, thermisch geringwirkend isolierte Seite (5) und dieser thermisch geringwirkend isolierten Seite (5) gegenüberliegend eine zweite, thermisch stark isolierte Seite mit einer eine thermischen Isolation (4) aufweist.

4. Wärmespendendes Produkt mit Wärmespeicher (1) und Temperatureinstellung nach einem der vorhergehenden Ansprüche, **dadurch gekennzeichnet, dass** zumindest eine zweite wärmeisolierende Abdeckschicht (3) vorgesehen ist, die über die erste wärmeisolierende Abdeckschicht (2) legbar oder anstelle der ersten wärmeisolierende Abdeckschicht (2) auf den Wärmespeicher (1) legbar ist.

5. Wärmespendendes Produkt mit Wärmespeicher (1) und Temperatureinstellung nach dem vorhergehenden Anspruch, **dadurch gekennzeichnet, dass** das die zweite wärmeisolierende Abdeckschicht (3) an der gleichen Seite mit dem Wärmespeicher (1) verbunden ist wie die erste wärmeisolierende Abdeckschicht (2).

6. Wärmespendendes Produkt mit Wärmespeicher (1) und Temperatureinstellung nach einem der vorhergehenden Ansprüche, **dadurch gekennzeichnet, dass** die erste wärmeisolierende Abdeckschicht (2) und die zweite wärmeisolierende Abdeckschicht (3) unterschiedliche Wärmeleitwerte aufweisen, wobei hierzu unterschiedlich wärmeleitende Materialien und/oder unterschiedliche Dicken der wärmeisolierende Abdeckschichten (2,3) eingesetzt sind.

7. Wärmespendendes Produkt mit Wärmespeicher (1) und Temperatureinstellung nach einem der vorhergehenden Ansprüche, **dadurch gekennzeichnet, dass** die wärmeisolierende Abdeckschicht (2) oder, im Falle mehrerer wärmeisolierender Abdeckschichten (2,3), zumindest die äußere wärmeisolierende Abdeckschicht (2 oder 3) an ihrer der Verbindung mit dem Wärmespeicher (1) abgewandten Seite ein Befestigungsmittel zur lösbaren Befestigung mit dem Wärmespeicher (1), insbesondere in Form einer längs des Randes verlaufenden Einstecktasche oder eines Haltemittels, aufweist.

8. Wärmespendendes Produkt mit Wärmespeicher (1) und Temperatureinstellung nach einem der vorhergehenden Ansprüche, **dadurch gekennzeichnet, dass** um den Wärmespeicher (1) ein hüllenartiger Überzug (6) vorgesehen ist, wobei zumindest eine wärmeisolierende Abdeckschicht (2,3) von wenigstens einem Abschnitt des hüllenartigen Überzugs (6) gebildet ist.

9. Wärmespendendes Produkt mit Wärmespeicher (1) und Temperatureinstellung nach dem vorhergehenden Anspruch, **dadurch gekennzeichnet, dass** der hüllenartige Überzug (6) an wenigstens zwei Seiten eine unterschiedliche Wärmeleitfähigkeit aufweist, so dass durch Drehen des Überzuges relativ zum Wärmespeicher (1) wahlweise ein Wärmeleitabschnitt (7) des hüllenartigen Überzugs mit einer thermisch geringen Isolationswirkung oder ein thermischer Isolierabschnitt (3) des hüllenartigen Überzugs mit einer thermisch hohen Isolationswirkung vor die wärmeabgebende Oberfläche bewegbar ist.

10. Wärmespendendes Produkt mit Wärmespeicher (1) und Temperatureinstellung nach Anspruch 6, **dadurch gekennzeichnet, dass** die erste wärmeisolierende Abdeckschicht (1) und die zweite wärmeisolierende Abdeckschicht (3) unterschiedliche Farben zur Kenntlichmachung der Wärmeleitfähigkeit aufweisen.

11. Wärmespendendes Produkt mit Wärmespeicher (1) und Temperatureinstellung nach Anspruch 9, **dadurch gekennzeichnet, dass** der Wärmeleitabschnitt (7) und/oder der thermische Isolierabschnitt (3) des hüllenartigen Überzugs optisch markiert sind.

12. Wärmespendendes Produkt nach einem der vorhergehenden Ansprüche, **dadurch gekennzeichnet, dass** ein Wärmeindikator vorgesehen ist, der derart mit dem Wärmespeicher (1) zusammenwirkt, dass er die Temperatur des Wärmespeichers (1) anzuzeigen vermag.

13. Wärmespendendes Produkt nach Anspruch 12, **dadurch gekennzeichnet, dass** der Wärmeindikator derart mit dem Wärmespeicher (1) verbunden ist, dass er die Temperatur des Wärmespeichers anzunehmen vermag.

14. Wärmespendendes Produkt nach einem der beiden vorhergehenden Ansprüche, **dadurch gekennzeichnet, dass** der Wärmeindikator derart ausgebildet ist, dass er bei Über- und Unterschreiten einer definierten Grenztemperatur jeweils durch einen Farbumschlag selbsttätig seine Farbe zu ändern vermag.

15. Wärmespendendes Produkt nach dem vorhergehenden Anspruch, **dadurch gekennzeichnet, dass** der Wärmeindikator einen thermochromen Farbstoff aufweist, der unter Ausschluss von Luft und Feuchtigkeit im Wärmeindikator eingekapselt ist.
